(19) Europäisches Patentamt
European Patent Office
Office européen des brevets

(11) Veröffentlichungsnummer: **0 256 504 B1**

(12) **EUROPÄISCHE PATENTSCHRIFT**

(45) Veröffentlichungstag der Patentschrift: **27.12.90**

(51) Int. Cl.[5]: **C07C 205/21**, C07C 205/25, C07C 205/26, C02F 1/26

(21) Anmeldenummer: **87111656.2**

(22) Anmeldetag: **12.08.87**

(54) **Verfahren zur Extraktion von Nitro-hydroxy-aromaten aus wässrigen Lösungen.**

(30) Priorität: **14.08.86 DE 3627653**

(43) Veröffentlichungstag der Anmeldung: **24.02.88 Patentblatt 88/8**

(45) Bekanntmachung des Hinweises auf die Patenterteilung: **27.12.90 Patentblatt 90/52**

(84) Benannte Vertragsstaaten: **BE CH DE ES FR GB IT LI NL**

(56) Entgegenhaltungen:
**DE-C- 406 150**
**US-A- 3 597 351**

(73) Patentinhaber: **HOECHST AKTIENGESELLSCHAFT, Postfach 80 03 20, D-6230 Frankfurt am Main 80(DE)**

(72) Erfinder: **Gössel, Helmut, Dr., Kopernikusstrasse 32, D-6230 Frankfurt am Main 80(DE)**
Erfinder: **Kuckertz, Herbert, Dr., Ludwig-Schäfer-Weg 11, D-6233 Kelkheim (Taunus)(DE)**
Erfinder: **Rittner, Siegbert, Dr., Kornblumenweg 5, D-6105 Mörfelden-Walldorf(DE)**
Erfinder: **Rosenfelder, Josef, Dr., Wingertstrasse 12a, D-6238 Hofheim am Taunus(DE)**
Erfinder: **Wojtech, Bernhard, Dr., Kelkheimer Strasse 67, D-6232 Bad Soden Am Taunus(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein Verfahren zur Extraktion von Nitro-hydroxy-aromaten aus wäßrigen Lösungen.

Es ist bekannt, daß bei der Herstellung von nitro-hydroxy-aromatischen Verbindungen große Mengen an Abwässern entstehen, die nur sehr schwierig gereinigt werden können. Nitro-hydroxy-aromaten sind toxisch und außerdem biologisch überwiegend schwer abbaubar, so daß die Entsorgung solcher Abwässer strengen behördlichen Vorschriften unterliegt. Nach dem heutigen Stand der Technik werden nitro-hydroxy-aromatische Abwässer vor der biologischen Reinigung meist einer Vorreinigung unterworfen, um die Konzentration zu vermindern und damit die Gefar zu verringern, daß die Mikroorganismen der biologischen Reinigung abgetötet werden. Zur Vorreinigung wurden bisher neben Oxydations- und Reduktionsverfahren vorwiegend solche Methoden vorgeschlagen, die nach den Prinzipien thermischer Trennverfahren wie z.B. Adsorption an Kohle arbeiten (Winnacker-Küchler, Chemische Technologie, 4. Auflage, Band 6, 1982, S. 173).

Oxydations- und Reduktionsverfahren, bei denen z.B. mit Ozon oxydiert oder mit Wasserstoff in Gegenwart von Edelmetall-Katalysatoren reduziert wird, sind recht aufwendige chemische Verfahren. Sie benötigen Hilfschemikalien und führen zu Verlusten, denn infolge des chemischen Abbaus ist eine Rückgewinnung der Nitro-hydroxy-aromaten nicht mehr möglich.

Kohleadsorptionsverfahren haben ebenfalls Schwächen. So können sie beispielsweise nur bei verdünnten nitrohydroxy-aromatischen Abwässern angewandt werden. Schädlich sind hierbei auch höhere anorganische Salzgehalte im Abwasser, die zu Porenverklebung der Aktivkohle führen. Der Kohleadsorption schließt sich in der Regel ein regenerativer Verfahrensschritt an, der normalerweise thermisch durchgeführt werden muß und darum apparativ aufwendig ist.

Ein weiteres allgemeines Adsorptionsverfahren zur Entfernung von phenolischen und/oder metallischen Verunreinigungen aus wäßrigen Lösungen wird in US-PS 3 597 351 beschrieben. Bei diesem Verfahren adsorbieren die Verunreinigungen an ein wasserunlösliches basisches Polymer.

Aus DE-OS 3 436 349 war nun bereits bekannt, daß man Phenole, die bekanntlich schwach sauer sind und teilweise reduzierend wirken, mit Hilfe von Salzen höherer Amine extrahieren kann.

Es wurde nun überraschenderweise gefunden, daß man die stark sauren und oxydierend wirkenden Nitro-hydroxyaromaten aus Abwässern sogar extrem weitgehend extrahieren kann, wenn man als Extraktionsmittel solche Salze verwendet. Gegenstand der Erfindung ist daher ein Verfahren zur Extraktion von Nitro-hydroxy-aromaten der allgemeinen Formel

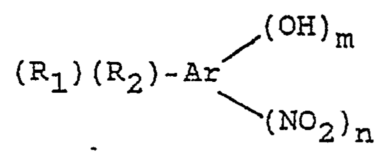

worin $R_1$ und $R_2$ Wasserstoff, Fluor, Chlor, Brom, einen Methyl-, Trifluormethyl-, Ethyl-, Propyl-, i-Propyl-, n-Butyl- oder sec. Butylrest, Ar ein Benzol- oder Naphthalinrest, und m, n 1, 2 oder 3 bedeuten, aus wäßrigen Lösungen, dadurch gekennzeichnet, daß man als Extraktionsmittel ein Aminsalz verwendet, das aus einem aliphatischen Amin mit einer Gesamtkohlenstoffzahl von 10 bis 75 und einer starken Säure besteht, wobei das-Aminsalz unverdünnt oder mit einem organischen Lösemittel verdünnt eingesetzt werden kann.

Das Aminsalz wird entweder separat hergestellt und dann dem Abwasser zugegeben, oder es wird in situ gebildet. Zur in situ-Bildung wird, wenn das Abwasser bereits genügend Säure enthält, im allgemeinen lediglich Amin zugegeben, andernfalls Amin und Säure.

Die den Aminsalzen zugrundeliegenden aliphatischen Amine, die primär, sekundär oder tertiär sein können, sollen eine Gesamtkohlenstoffzahl von 10 bis 75, vorzugsweise 20 bis 50 C-Atomen haben und können geradkettig, cyclisch oder verzweigt sein. Besonders bevorzugt unter den genannten Aminen sind wegen ihrer geringen chemischen Reaktivität die tertiären Amine, vor allen Tri-n-octylamin, Tri-isooctylamin, Tri-n-decylamin, Tri-isodecylamin, Tr-n-dodecylamin, Tri-isododecylamin bzw. deren Gemische.

Als starke Säuren zur Aminsalzbildung eignen sich besonders Mineralsäuren, wie Phosphorsäure, Salpetersäure, Salzsäure oder Schwefelsäure, vor allem Salzsäure oder Schwefelsäure.

Das Aminsalz bildet sich schnell und quantitativ beim Vermischen des wasserunlöslichen Amins mit einem sauren Abwasser, wobei die Säure aus der wäßrigen Phase unter Ionenpaarbildung in die organische Phase übergeht. Das Gleichgewicht dieser "Neutralisation" liegt ganz auf der Seite des Aminsalzes. Die Gleichgewichtskonstanten betragen $10^4$ bis $10^8$, je nach dem Amin und der Säure. Solche Aminsalze haben die Zusammensetzung $(RH_2NH)X$, $(R_2HNH)X$, $(R_3NH)X$, wobei X das Anion der Säure ist.

Werden die Aminsalze separat hergestellt, so können sie unverdünnt dem Abwasser zugegeben werden, sie können aber zur Viskositätsverminderung auch mit einem organischen Lösemittel, z.B. einem

Kohlenwasserstoff, verdünnt sein. Auch eine Teilumwandlung des Amins in das Aminsalz ist möglich, wobei das restliche (freie) Amin als Verdünnungsmittel wirkt. Analog kann bei der in situ-Bildung der Aminsalze ein organisches Lösemittel anwesend sein.

Als Beispiele seien genannt:

2-Nitrophenol, 3-Nitrophenol, 4-Nitrophenol, 2-Nitro-naphthol-(1), 4-Nitro-naphthol-(1), 1-Nitro-naphthol-(2), 2-Chlor-3-nitrophenol, 2-Chlor-4-nitrophenol, 2-Chlor-5-nitrophenol, 3-Chlor-2-nitrophenol, 3-Chlor-4-nitrophenol, 3-Chlor-5-nitrophenol, 4-Chlor-2-nitrophenol, 4-Chlor-3-nitrophenol, 5-Chlor-2-nitrophenol, 6-Chlor-2-nitrophenol, 2,6-Dichlor-4-nitrophenol, 4,6-Dichlor-2-nitrophenol, 3-Nitro-o-kresol, 4-Nitro-o-kresol, 5-Nitro-o-kresol, 6-Nitro-o-kresol, 2-Nitro-m-kresol, 4-Nitro-m-kresol, 5-Nitro-m-kresol, 6-Nitro-m-kresol, 2-Nitro-p-kresol, 3-Nitro-p-kresol, 2,4,6-Trinitro-m-kresol, 2,3,6-Trichlor-6-nitrophenol, 2,4-Dibrom-6-nitrophenol, 2,6-Dibrom-4-nitrophenol, 3-Trifluormethyl-4-nitrophenol, 4-Trifluormethyl-2-nitrophenol, 4-Trifluormethyl-2,6-dinitrophenol, 4-Trifluormethyl-3-chlor-2,6-dinitrophenol, 2,3-Dinitrophenol, 2,4-Dinitrophenol, 2,5-Dinitrophenol, 2,6-Dinitrophenol, 2,4-/2,6-Dinitrophenol-Gemische, 3,4-Dinitrophenol, 3,5-Dinitrophenol, 2,4-Dinitroresorcin, 3,5-Dinitro-resorcin, Dinitro-o-kresol (= 2-Hydroxy-3,5-dinitro-toluol), Dinitro-sec.-butyl-phenol (= 2-Hydroxy-3,5-dinitro-butyl-benzol), 1,3,5-Trinitrophenol.

Die Konzentration der zu extrahierenden Nitro-hydroxy-aromaten in der wäßrigen Phase kann bis zur Sättigungskonzentration reichen. Auch können die wäßrigen Lösungen andere gelöste organische oder anorganische Verbindungen und suspendierte Teilchen enthalten, sofern diese die Extraktion nicht stören. Das Verfahren erlaubt auch die Extraktion der Nitro-hydroxy-aromaten aus sehr verdünnten Lösungen.

Der große Vorteil des erfindungsgemäßen Verfahrens liegt nicht nur in seinem breiten Anwendungsbereich, sondern vor allem in seiner extrem hohen Reinigungswirkung, die sich in Verteilungskoeffizienten bis über 10 000 äußert. Dadurch ist es möglich, verunreinigtes Abwasser bereits in einer Stufe, d.h. durch einmaliges einfaches Ausrühren mit dem Extraktionsmittel weitgehend, bis auf wenige ppm oder darunter bis in den ppb-Bereich, zu reinigen. Der Extraktionsaufwand ist aber nicht nur wegen der einstufigen Verfahrensweise so gering, sondern das hohe Extraktionsvermögen erlaubt es auch, mit sehr kleinen Extraktionsmittelmengen auszukommen, so daß das Phasenvolumenverhältnis Abwasser/Extraktionsmittel bis auf 20:1 eingestellt werden kann. Bei mehrstufiger Fahrweise sind sogar noch höhere Werte möglich. Das einstufige Ausrühren kann diskontinuierlich oder (vorteilhafter) kontinuierlich geschehen. Führt man die Extraktion mehrstufig im Kreuzstrom oder kontinuierlich im Gegenstrom aus, so erreicht man auch bei höheren Konzentrationen an Nitro-hydroxy-aromaten im Abwasser den ppb-Bereich ohne Schwierigkeiten.

Ein anderer Vorteil des erfindungsgemäßen Verfahrens ist bei mineralsäurehaltigen Abwässern neben der Entfernung der Nitro-hydroxy-aromaten eine Abtrennung der Mineralsäure, die aus dem Abwasser in die Aminphase unter Bildung des extraktionswirksamen Aminsalzes übergeht.

Ein weiterer Vorteil des erfindungsgemäßen Verfahrens ist die Möglichkeit der einfachen Rückgewinnung des Amins aus dem Extrakt durch einstufige Rückextraktion mit Natronlauge. Dabei gehen die Nitro-hydroxy-aromaten zusammen mit der am Amin gebundenen Mineralsäure quantitativ als Natriumsalze in die wäßrige NaOH-Phase, während das freie in organischen Phase bleibt und wieder in die Abwasserextraktion zurückgeführt werden kann. Die Amin-Rückgewinnung läßt sich einstufig ebenfalls durch einfaches Ausrühren erreichen, wenn eine dem Gehalt an Mineralsäure + Nitro-hydroxy-aromat äquivalente NaOH-Menge eingesetzt wird. Vorzugsweise verwendet man konzentrierte Natronlauge in äquivalenter Menge, weil dann eine besonders hohe Konzentration der Nitro-hydroxy-aromaten im Rückextrakt erzielt wird. Nach der Anreicherung im Rückextrakt, die eine bis zwei Zehnerpotenzen betragen kann, können die Nitro-hydroxy-aromaten wiedergewonnen werden, entweder als Salze oder nach dem Ansäuern in der Hydroxyform, wobei sie zum größten Teil ausfallen und abfiltriert werden können. Auf diese Weise ist ein praktisch verlustfreies Arbeiten gewährleistet.

Die Erfindung soll anhand folgender Beispiele erläutert werden. Die Extraktionen wurden dabei immer bei Raumtemperatur ausgeführt. Der Name Hostarex A 327 ( in der BRD) als Warenzeichen geschützt) bezeichnet ein Gemisch aus gleichen Teilen Tri-n-octylamin und Tri-n-decylamin.

Vergleichsbeispiel

1 000 ml einer wäßrigen 0,50 Gew.-prozentigen 4-Nitrophenol-Lösung wurden mit 100 ml (80,6 g) Hostarex A 327 in einem Schüttelzylinder ins Gleichgewicht gesetzt und die Phasen nach ihrer Trennung analysiert. Die wäßrige Phase (995,7 g) hatte eine Konzentration von 576 ppm 4-Nitrophenol, die Extrakt-(Amin-) phase (84,9 g) eine Konzentration von 5,21 Gew.-% 4-Nitrophenol. Das ergibt einen Verteilungskoeffizienten D (= Konzentrationsverhältnis organische Phase zu wäßriger Phase) von 90,5.

Beispiel 1

1 000 ml der gleichen wäßrigen Lösung wie im Vergleichsbeispiel mit 0,50 Gew.-% 4-Nitrophenol wurde erst mit 22 g konzentrierter Salzsäure versetzt (0,80 Gew.-% in der Lösung) und dann wie im Vergleichsbeispiel mit 100 ml (80,6 g) Hostarex A 327 ins Gleichgewicht gesetzt. Die wäßrige Phase (1 003, 4 g) enthielt nur noch 7,6 ppm 4-Nitrophenol, die Aminphase (99,2 g) dagegen 5,035 Gew.-%. Das entspricht einem Verteilungskoeffizienten (D) von 6 625.

Beispiel 2

Die Reinigungswirkung verschiedener Amine wurde an Proben eines Abwassers mit einem Gehalt von 0,702 Gew.-% 4-Nitrophenol und 1,4 Gew.-% Salzsäure untersucht. Dazu wurden die Verteilungsgleichgewichte durch Ausrühren in einem Rührgefäß jeweils bei einem Volumenverhältnis von Abwasser:Amin = 10:1 bestimmt. Als Extraktionsmittel wurden eingesetzt:

A: Hostarex A 327
B: Tri-iso-octylamin (=Hostarex A 324, in der BRD als Warenzeichen geschützt)
C: Di-2-ethylhexylamin.

Die Gleichgewichtskonzentrationen in den einzelnen Phasen mit den entsprechenden Verteilungskoeffizienten (D) und Extraktionsausbeuten zeigt die folgende Tabelle 1:

Tabelle 1:

| Extraktionsmittel | 4-Nitriphenol-Aminphase Gew.-% | Kenzentration Abwasserphase ppm | D | Extraktionsausbeute % |
|---|---|---|---|---|
| A | 7,195 | 6,6 | 10 902 | 99,99 |
| B | 6,975 | 7,1 | 9 824 | 99,98 |
| C | 6,840 | 48,4 | 1 413 | 99,93 |

Beispiel 3

1 000 g Hostarex A 327 wurden durch intensives Rühren mit 443,5 ml 4n-Schwefelsäure in Kontakt gebracht. Dabei ging die Schwefelsäure quantitativ aus der wäßrigen Phase in die organische Aminphase über, so daß diese zu 70 % in das Aminsulfat $(R_3NH)_2 SO_4$ umgewandelt wurde. Nach der Abtrennung der wäßrigen Phase wurde dieses Aminsulfat-Amin-Gemisch als Extraktionsmittel eingesetzt, um aus einer wäßrigen Lösung, die außer 0,50 Gew.-% 4-Nitrophenol keine weiteren Bestandteile enthielt, das Nitrophenol zu entfernen. Bei einem Phasenvolumenverhältnis Abwasser: Extraktionsmittel von 10:1 wurden durch einstufiges Ausrühren im Rührgefäß folgende Phasenkonzentrationen an 4-Nitrophenol erhalten: Extrakt-(Amin-)Phase 5,458 Gew.-%, wäßrige Phase 4,5 ppm. Das entspricht einem Verteilungskoeffizienten von 12 130 und einer Extraktionsausbeute von 99,91 %.

Beispiel 4

Bestimmt wurde das Extraktionsverhalten von Aminen, die durch Lösemittel verdünnt waren. Dazu wurde Hostarex A 327 einmal mit n-Dodekan und einmal mit ®Solvesso 150 ($C_{10}$-$C_{11}$ Alkylbenzole) zu einer 40 Gew.-%igen Aminlösung (=Extraktionsmittel) verdünnt. Das Abwasser enthielt 0,556 Gew.-% 4-Nitrophenol und 1,32 Gew.-% Salzsäure. Die Gleichgewichtseinstellung erfolgte einstufig in einem Rührgefäß bei einem Phasenvolumenverhältnis Abwasser:Extraktionsmittel von 4:1. Die folgende Tabelle 2 zeigt die Ergebnisse.

Tabelle 2:

| Extraktionsmittel | 4-Nitrophenol-Aminphase Gew.-% | Kenzentration Abwasserphase ppm | D | Extraktionsausbeute % |
|---|---|---|---|---|
| 40 Gew.-% Hostarex A 327 in n-Dodekan | 2,886 | 8,5 | 3 400 | 99,85 |
| 40 Gew.-% Hostarex A 327 in Solvesso 150 | 2,522 | 4,8 | 5 250 | 99,91 |

Beispiel 5

Ein Abwasser mit einem Gehalt von 0,702 Gew.-% 4-Nitrophenol und 1,4 Gew.-% Salzsäure wurde in einer 3-stufigen Kreuzstromextraktion mit Hostarex A 327 bei einem Phasenvolumenverhältnis Abwasser:Amin von 20:1 je Stufe gereinigt. Die 4-Nitrophenolkonzentration im Abwasser betrug nach der 1. Stufe 19,1 ppm, nach der 2. Stufe 0,7 ppm und nach der 3. Stufe weniger als 0,1 ppm.

Beispiel 6

Das gleiche Abwasser wie im Beispiel 4 wurde einstufig in einem Rührgefäß mit Hostarex A 327 bei einem Phasenvolumenverhältnis Abwasser:Amin von 10:1 (1 000 ml: 100 ml) gereinigt. Der Extrakt (102.2 g) enthielt 7,19 Gew.-%, das gereinigte Abwasser (1 025,6 g) 7 ppm 4-Nitrophenol. Das entspricht einer Extraktionsausbeute von 99,9 %. Nach der Phasentrennung wurde zur Rückextraktion der Extrakt einstufig mit 56 g 15 %iger Natronlauge ausgerührt. Dabei gingen das 4-Nitrophenol und die am Amin gebundene Salzsäure vollständig als Na-Salze in die Natronlaugephase über, während das regenerierte und gereinigte Hostarex A 327 wieder in die Extraktion zurückgeführt wurde.

Beispiel 7

Ein Abwasser mit einem Gehalt von 0,179 Gew.-% 2-Nitrophenol und 0,742 Gew.-% Salzsäure wurde mit Hostarex A 327 im Volumenverhältnis Abwasser:Amin von 10:1 (600 ml:60 ml) einstufig in einem Rührgefäß bis zur Gleichgewichtseinstellung ausgerührt. Der Extrakt (56,36 g) hatte eine Konzentration von 2,019 Gew.-%, das Abwasserraffinat (629,8 g) eine Konzentration von 6,4 ppm 2-Nitrophenol. Das entspricht einem Verteilungskoeffizienten von 3 150 und einer Extraktionsausbeute von 99,65 %. Die Rückextraktion erfolgte einstufig durch Ausrühren des Extrakts mit 33 g 15 %iger Natronlauge. Dabei traten das 2-Nitrophenol und die am Amin gebundene Salzsäure vollständig als Natriumsalze in die Natronlaugephase über. Das regenerierte und gereinigte Hostarex A 327 wurde in die Extraktion zurückgeführt.

Beispiel 8

Ein Abwasser mit einem Gehalt von 307 ppm 2,4-Dinitrophenol und 1,5 % Salzsäure wurde einstufig mit Hostarex A 327 im Volumenverhältnis Abwasser:Amin von 20:1 (600 ml:30 ml) bis zur Gleichgewichtseinstellung ausgerührt. Der Extrakt (29,5 g) enthielt 0,650 Gew.-%, das Abwasserraffinat (618,7 g) 0,5 ppm 2,4-Dinitrophenol. Das entspricht einem Verteilungskoeffizienten von 13 000 und einer Extraktionsausbeute von 99,98 %. Die vollständige Abtrennung des Dinitrophenols und der Salzsäure aus dem Extrakt erfolgte durch einstufige Rückextraktion mit 10 g 25 Gew.-%iger Natronlauge.

Beispiel 9

Ein Abwasser mit einem Gehalt von 180 ppm 4-Chlor-2-nitrophenol und 0,86 Gew.-% Salzsäure wurde einstufig mit Hostarex A 327 im Volumenverhältnis Abwasser:Amin von 10:1 (650 ml:65 ml) bis zur Gleichgewichtseinstellung ausgerührt. Der Extrakt (60,4 g) enthielt 0,196 Gew.-%, das Abwasserraffinat (650,0 g) 0,2 ppm 4-Chlor-2-nitrophenol. Das entspricht einem Verteilungskoeffizienten von 9 800 und einer Extraktionsausbeute von 99,89 %. Zur vollständigen Rückextraktion durch einstufiges Ausrühren wurden 22 g einer 25 %igen Natronlauge benötigt.

Beispiel 10

Ein Abwasser mit einem Gehalt von 394 ppm 4-Methyl-2-nitrophenol und 1,12 Gew.-% Salzsäure wurde einstufig mit Hostarex A 327 im Volumenverhältnis Abwasser:Amin von 10:1 (600 ml:60 ml) bis zur Gleichgewichtseinstellung ausgerührt. Der Extrakt (55,52 g) enthielt 0,431 Gew.-%, das Abwasserrraffinat (603,24 g) 2,3 ppm 4-Methyl-2-nitrophenol. Das entspricht einem Verteilungskoeffizienten von 1 874 und einer Extraktionsausbeute von 99,42 %. Zur vollständigen Rückextraktion durch zweistufiges Ausrühren wurden 33 g einer 15 Gew.-%igen Natronlauge benötigt.

Beispiel 11

Ein Abwasser mit einem Gehalt von 0,297 Gew.-% 3-Trifluormethyl-4-nitrophenol und 1,3 Gew.-% Salzsäure wurde einstufig mit Hostarex A 327 im Volumenverhältnis Abwasser:Amin von 10:1 (600 ml:60 ml) bis zur Gleichgewichtseinstellung ausgerührt. Der Extrakt (58,82 g) enthielt 3,069 Gew.-%, das Abwasserraffinat (597,31 g) 0,2 ppm 3-Trifluormethyl-4-nitrophenol. Das entspricht einem Verteilungskoeffizienten von 153 400 und einer Extraktionsausbeute von über 99,99 %. Zur vollständigen Rückextraktion durch einstufiges Ausrühren wurden 35 g einer 15 Gew.-%igen Natronlauge benötigt.

Beispiel 12

Ein Abwasser mit einem Gehalt von 0,150 Gew.-% 2,4,6-Trinitrophenol (Pikrinsäure) und 1,3 Gew.-% Salzsäure wurde einstufig mit Hostarex A 327 im Volumenverhältnis Abwasser:Amin von 10:1 (1000 ml:100 ml) bis zur Gleichgewichtseinstellung ausgerührt. Der Extrakt (99,21 g) enthielt 1,529 Gew.-%, das Abwasserraffinat (992,7 g) 0,3 ppm Pikrinsäure. Das entspricht einem Verteilungskoeffizienten von 50 970 und einer Extraktionsausbeute von 99,98 %. Zur vollständigen Rückextraktion durch einstufiges Ausrühren wurden 56 g einer 15 Gew.-%igen Natronlauge benötigt.

## Patentansprüche

1. Verfahren zur Extraktion von Nitro-hydroxy-aromaten der allgemeinen Formel

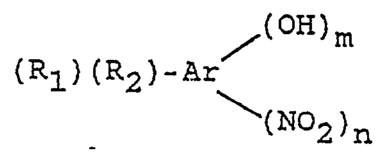

worin $R_1$ und $R_2$ Wasserstoff, Fluor, Chlor, Brom, einen Methyl-, Trifluormethyl-, Ethyl-, Propyl-, i-Propyl-, n-Butyl- oder sec.-Butylrest, Ar ein Benzol- oder Naphthalinrest, und m, n 1, 2 oder 3 bedeuten, aus wäßrigen Lösungen, dadurch gekennzeichnet, daß man als Extraktionsmittel ein Aminsalz verwendet, das aus einem aliphatischen Amin mit einer Gesamtkohlenstoffzahl von 10 bis 75 und einer starken Säure besteht, wobei das Aminsalz unverdünnt oder mit einem organischen Lösemittel verdünnt eingesetzt werden kann.

2. Verfahren nach Anspruch 1, dadurch gekennzeichnet, daß man ein Salz eines tertiären Amins verwendet.

3. Verfahren nach Anspruch 1 oder 2, dadurch gekennzeichnet, daß man ein Aminsalz verwendet, das von der Salzsäure oder Schwefelsäure abgeleitet ist.

## Claims

1. A process for extracting nitrohydroxyaromatics of the general formula

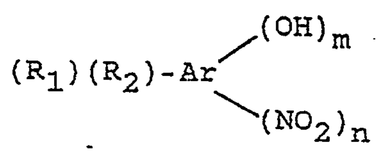

where $R_1$ and $R_2$ denote hydrogen, fluorine, chlorine, bromine, or a methyl, trifluoromethyl, ethyl, propyl, i-propyl, n-butyl or sec.-butyl radical, Ar denotes a benzene or naphthalene radical, and m and n denote 1, 2 or 3, from aqueous solutions, which comprises using as the extracting agent an amine salt which comprises an aliphatic amine having a total carbon number of 10 to 75 and a strong acid, it being possible for the amine salt to be employed undiluted or diluted with an organic solvent.

2. The process as claimed in claim 1, wherein a salt of a tertiary amine is used.

3. The process as claimed in claim 1 or 2, wherein an amine salt which is derived from hydrochlorid acid or sulfuric acid is used.

## Revendications

1. Procédé pour extraire des composés nitro-hydroxyaromatiques de formule générale:

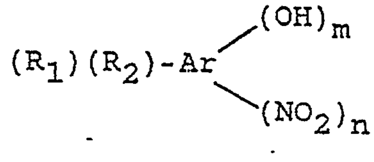

(dans laquelle $R_1$ et $R_2$ representent chacun un atome d'hydrogène, de fluor, de chlore, de brome, un reste méthyle, trifluorométhyle, éthyle, propyle, isopropyle, butyle ou sec.-butyle; Ar représente un reste benzénique ou naphtalénique, et m et n valent chacun 1, 2 ou 3) de solutions aqueuses, procédé caractérisé en ce qu'on utilise comme agent d'extraction un sel d'amine qui consiste en une amine aliphatique

ayant un nombre total d'atomes de carbone de 10 a 75 et en un acide fort, le sel d'amine pouvant etre utilisé sans etre dilué ou bien en étant dilué à l'aide d'un solvant organique.

2. Procédé selon la revendication 1, caractérisé en ce qu'on utilise un sel d'une amine tertiaire.

3. Procédé selon la revendication 1 ou 2, caractérisé en ce qu'on utilise un sel d'amine qui dérive de l'acide chlorhydrique ou de l'acide sulfurique.